# EUROPEAN PATENT APPLICATION

(11) **EP 2 772 274 A1**
(43) Date of publication of application: **03.09.2014**
(21) Application number: 12842868.7
(22) Date of filing: 17.01.2012
(51) Int. Cl.: A61L 27/36, A61L 27/60, A61F 2/24, A61F 2/14, A61F 2/08, A61F 2/02

(54) **METHOD FOR TREATING ANIMAL-DERIVED COLLAGEN FIBRE MATERIALS**

(30) Priority: 25.10.2011 CN 201110327475
(71) Applicant: Shanghai MicroPort Medical (Group) Co., Ltd., Pudong New Area Shanghai Shanghai 201203 (CN)
(72) Inventor: CHEN, Dakai, Shanghai 201203 (CN); LI, Yu, Shanghai 201203 (CN); FANG, Yuan, Shanghai 201203 (CN); TIAN, Cong, Shanghai 201203 (CN); DONG, Jiaoming, Shanghai 201203 (CN); ZHOU, Ling, Shanghai 201203 (CN); LIU, Xiang, Shanghai 201203 (CN); CHEN, Guoming, Shanghai 201203 (CN); YUE, Chengyun, Shanghai 201203 (CN); LUO, Qiyi, Shanghai 201203 (CN)
(74) Representative: Modiano, Micaela Nadia
(86) International application number: PCT/CN2012/070482
(87) International publication number: WO 2013/060103

(57) **Abstract**

Provided is a method for treating animal-derived collagen fibre materials. The method comprises treating the animal-derived collagen fibre materials with a salt solution containing polyethylene glycol and an oxidant. After treatment by the method, the animal-derived collagen fibre materials are transplanted into a recipient, after which no immune inflammatory response occurs.

## Description

### FIELD OF THE INVENTION

The present invention relates to the field of medical device. More particularly, the present invention relates to a method for treating animal-derived collagen fibre materials.

### BACKGROUND ARTS

Animal-derived collagen fibre materials include tendon, cornea, cardiac valve, ligament, skin and diaphragm, etc., mainly consist of collagen fibres, elastic fibers and proteoglycans. Elastic fibrous layer is reticularly distributed in the whole layer of pericardium, collagen fibres represent a wavy and multilayered structure. Heart valve (cardiac valve) consists of pericardium components from animals, mainly falling into serosal layer, fibrous layer and connective tissue layer of external pericardium, it is one of animal-derived collagen fibre materials.

Bioprosthesis heart valve is the biomaterial used to treat heart diseases, compared with mechanical valve, it has the advantages of no need for long-term anticoagulation and having good hemodynamic performance, etc., although its shelf-life is still relatively low, its role in clinical application can't be replaced by mechanical valves. Currently, the usage rate of bioprosthetic valves has already exceeded that of mechanical valves abroad. Howerver, since there are immune responses in transplantations, bioprosthetic valves need special processing in advance.

There are several methods in the prior art to treat xenogeneic biovalve as follows:
First, glutaraldehyde fixation and chemical modification. Glutaraldehyde treatment can make collagen fibre crosslink to increase the stability of tissues, reduce immunogenicity, contributing to improve its in vitro mechanical property and durability; but at the same time, a great deal of soluble proteins are lost, its exposed amino acid residues have changed, phosphate bonds of collagens are exposed, providing a similar chemical environment to stimulate calcification (Golomb G; Ezra V Govalent binding of protamine by glutaraldehyde to bioprosthetic tissue:characterization and anticalcification effect 1992 (01)). Thus, based on glutaraldehyde fixation, various modification techniques have been developed and applied: treatment with a detergent or surface modifier, covalent binding of diphosphates, α-oleic acid treatment to prevent calcification, treatment with toluidine blue etc. These methods delay valvular tissue fatigue and the occurrence of calcification, but they can't resolve the issue fundamentally.

The second method is acellular bioprosthetic valve. The cells of original tissues are removed, a matrix is constructed, allowing the cells of a recipient to grow theron after being transplanted into a human body. Specific methods are: 1) decellularization (killing cells by hypoosmotic shock and nuclease treatment) (Goldstein, U.S. Pat. Nos. 5613982; 5632778; 5899936 and 5843182); 2) controlled cell autolysis (Jaffe, U.S. Pat. Nos. 5843180; 5843181 and 5720777); 3) radiation (Schinstein, U.S. Pat. Nos. 5795790; 8843431; 5843717 and 5935849) (Badylak, U.S. Pat. No. 6126686); 4) acid treatment (Abraham, U.S. Pat. No. 5993833). After being transplanted into a human body, human cells are differentiated into endothelial cells thereon in an in-vivo environment, finally, the materials will be remodeled into tissues similar to natural ones.

The third method is in vitro endothelialization. Valve materials are implanted after being decellularized, autologous cells are cultured, allowing the reendothelialization of exposed regions of bioprosthetic valve (Goldstein, U.S. Pat. No. 7318998). The effect of such bioprosthetic valve in preliminary clinical application is not satisfactory. In 2003, the first case of decellularized porcine biological tissue-engineered heart valve, Synergrafte™ (Cryolife Inc., USA) was applied in a clinical operation in Europe, but valve tearing, calcification and serious degeneration occurred in a short time after the operation (Simon P. Kasimir MT. Seebacher G et al. Barly failure of the tissue engineered porcine heart valve SYNERGRAFT in pediatric patients European Journal of Cardio-Thoracic Surgery. Volume 23, Issue 6, June 2003, Pages 1002-1006).

Since the molecular mechanism of stem cell differentiation is not clear and directed differentiation process can't be fully controlled, there are still many problems to be solved regarding in vitro endothelialization of bioprosthetic valve.

For the above defects, so far there is no satisfactory solution in bioprosthetic valve, and there is still room for further improvement.

The person skilled in the art will appreciate that the major cause of immune response is the difference in surface molecule between donor and recipient cells. If the implant contains living cells from donor, immune response will be enhanced; if the implant doesn't contain living cells, materials such as residual proteins still have immunogenicity. To remove these materials, potent means of chemical treatment have to be used in order to ensure that all these materials are removed. While during this process, tissue matrices are also destroyed.

Polyethylene glycol (PEG) is the compound with low toxicity and no immunogenicity, having good biocompatibility, which will not be degraded in vivo to generate toxic derivatives and rarely affect biological properties of the modified materials. It has been found that polyethylene glycol (PEG) acts on the implant, capable of reducing the immune response of recipient ("Heart Preservation Solution Containing Polyethylene Glycol: An Immunosuppressive Effect" by Collins, et al. in Lancet, 338:390 (1991)). In the mice liver transplantation assay conducted by Tokunaga, et al., the mean survival time of mice from test group in which implanted tissues were treated with PEG increased from 9.6 days to 11.9 days ("The Immunosuppressive Effect of Polyethylene Glycol in a Flush Solution for Rat Liver Transplantation" by Tokunaga , et al. in Transplantation, 54:756 8 (1992)).

It has also been found that histocompatibility antigen is a major determinant for generating transplant rejection. When receiving implants from different species or different individuals, the human immune system recognizes foreign tissues, produces inflammatory cells such as macrophage, secretes particles containing enzymes, and digests foreign materials. While more inflammatory cells will be produced during the digestion process until the foreign materials are removed. This digestion process is an oxidation reaction, thus inflammatory response is related with the oxidable degree of implant.

However, the prior art doesn't teach a method for treating bioprosthetic valve with PEG combined with an oxidant and reducing inflammatory response.

### SUMMARY OF THE INVENTION

The present invention provides a method for treating the animal-derived collagen tissue materials, allowing them to be transplanted into a recipient, after which no immune inflammatory response occurs.

To reduce the oxidable degree of the implant and prevent inflammatory response, hydrogen peroxide is used as the oxidant in the present invention. The naturally occurring oxidant during the human inflammatory response process is also hydrogen peroxide, which can react with a variety of groups such as aldehyde group, amino group, lipid, etc., to make them oxidated. In the oxidation reaction, tissue fibers need protecting to avoid degeneration and thus being destroyed. It has already been known that in a high-concentration salt solution, collagen molecule and animal fiber can keep stable, thus the oxidation reaction according to the present invention is performed in the high-concentration salt solution to protect tissue fibers.

Connective tissue mainly consists of collagen, elastic fiber, collagen protein, proteoglycan, lipid, nucleic acid, small soluble molecules, inorganic substance, water, etc. After a cell dies, small molecules are released, while macromolecules such as protein, proteoglycan and nucleic acid are difficult to be removed. These macromolecules can be precipitated by a high-concentration salt solution, meanwhile the interaction between proteoglycan and nucleic acid is destroyed by the solution, making them no longer adhere to collagen.

In the process of removing non-collagen materials and oxidation reaction, in order to keep collagen fibre intact, polyethylene glycol is added to the solution to prevent collagen fibre from dissolving.

Particularly, the present invention relates to a method for treating animal-derived collagen fibre materials, wherein, the animal-derived collagen fibre materials are treated with a salt solution containing polyethylene glycol and an oxidant.

According to the present invention, the concentration of salt solution is at least 2.5mol/L, preferably 2.5mol/L to 4.5mol/L.

According to the present invention, the oxidant is preferably hydrogen peroxide.

According to the present invention, the animal-derived collagen fibre materials are tendon, cornea, cardiac valve, ligament, skin and diaphragm, preferably bioprosthetic valve.

The method according to the present invention comprises the following steps:
Triming the tissue material removed from an animal body in a physiological saline, then soaking the tissue material in a stabilizing solution at 0 to 10°C, wherein the stabilizing solution is a physiological saline or Ethanol-30-70% (preferably 40-60%, more preferably 50%), and wherein the soaking is carried out not more than 48 hours when using the physiological saline and not more than 30 days when using the Ethanol;

Treating thus obtained tissue material with Solution I for 2 to 2400 hours at 0 to 10°C, wherein the Solution I comprises a polyethylene glycol having a molecular weight of 2000D to 20000D present at a concentration of 1% to 15%, sodium chloride present at a concentration of 2.5mol/L to 4.5mol/L, a phosphate buffer with pH 6.5 to 7 present at a concentration of 0.02 to 0.1 mol/L, an oxidant present at a concentration of 0.1% to 2%;

Removing the tissue material from Solution I and washing it with an aqueous solution of low concentration ethanol (e.g., no more than 60%) to remove Solution I.

The method according to the present invention further comprises after treating with the Solution I and washing with the aqueous solution of low concentration ethanol:
Treating the tissue material with a Solution II comprising Ethanol-30%-70% (preferably 40%-60%, more preferably 50%) and an anti-inflammatory drug present at a concentration of 10 to 200mg/L;

Optionally soaking the tissue material in a Solution III comprising Ethanol-30%-70% (preferably 40%-60%, more preferably 50%) and an anticoagulant present at a concentration of 100 to 1000 IU/ml;

Finally washing the tissue material with a physiological saline and storing in Ethanol-30%-70% (preferably 40%-60%, more preferably 50%) at 0 to 6°C.

Alternatively, the method according to the present invention further comprises after treating with the Solution I and washing with the aqueous solution of low concentration ethanol:
Soaking the tissue material in a Solution IV comprising Ethanol-30%-70% (preferably 40%-60%, more preferably 50%), an anti-inflammatory drug present at a concentration of 10 to 200mg/Land an anticoagulant present at a concentration of 100 to 1000 IU/ml;

Finally washing the tissue material with a physiological saline and storing in Ethanol-30%-70% (preferably 40%-60%, more preferably 50%) at 0 to 6°C.

In a preferred embodiment of the present invention, the anti-inflammatory drug is selected from the group consisting of indometacin, dexamethasone acetate, dexamethasone, rapamycin, prednisone, prednisone acetate, antiflamison, ibuprofen piconol, ibuprofen and bufexamac, while the anticoagulant is selected from the group consisting of heparin, enoxaparin and hirudin.

In addition to cardiac valve, other animal-derived collagen fibre materials, such as tendon, cornea, ligament, skin and diaphragm, etc., also consist of collagen fibre, elastic fiber and proteoglycan as the main components, therefore, the person skilled in the art can expect that the treatment method for valve materials used by present invention is equally suitable for animal-derived collagen fibre materials.

### ILLUSTRATION

In order to describe the present invention more clearly, a brief explanation with reference to the drawings is provided as follows. Obviously, the drawings are only some of the specific embodiments described by the present application. The method according to the present invention includes, but is not limited to these drawings.

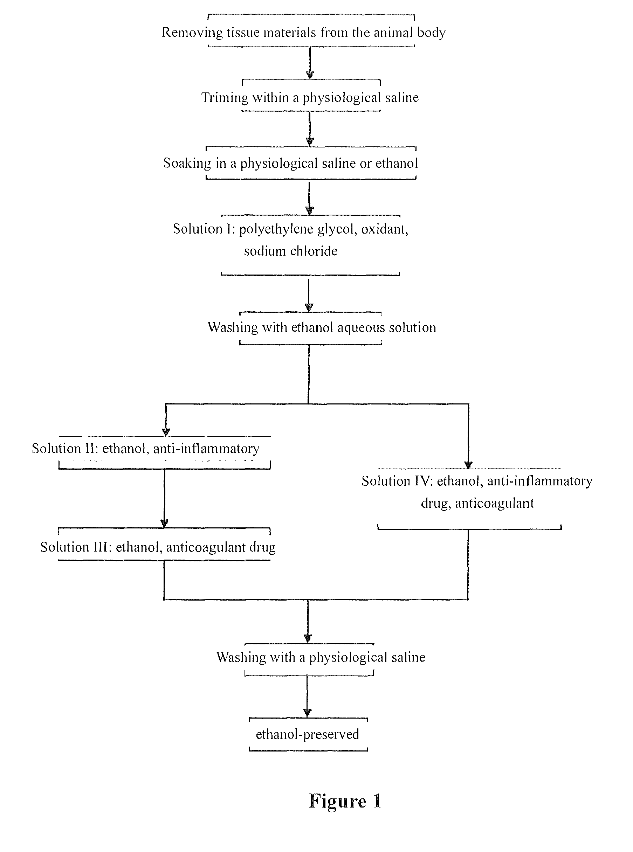

Figure 1 is the processing flow chart of animal-derived collagen fibre materials.
Figure 2 is the comparison of calcium content 35 days after bovine pericardium biovalve, treated by Example 1, Example 2 and Example 3 of the present invention and glutaraldehyde, is transplanted into mice.

### DETAILED DESCRIPTION OF THE INVENTION

For a further understanding of the invention, the preferred embodiments of the present invention will be described with the following examples. This description is only an example of the characteristics and advantages of the present invention, not to limit the claimed scope of the invention.

### Example 1

1. Soaking the bioprosthetic valve in an Ethanol-50% solution for 10 days.
2. Putting the bioprosthetic valve treated in step 1 into a solution for 80 hours, wherein the solution comprises a polyethylene glycol having a molecular weight of 2000D present at a concentration of 5%, sodium chloride present at a concentration of 2.5mol/L, a phosphate buffer with pH 7 present at a concentration of 0.04 mol/L and hydrogen peroxide present at a concentration of 1%.

### Example 2

1. Soaking the bioprosthetic valve in Ethanol-50% solution for 20 days.
2. Treating the bioprosthetic valve treated in step 1 in a solution for 90 hours, wherein the solution comprises a polyethylene glycolhaving a molecular weight of 6000D present at a concentration of 5%, sodium chloride present at a concentration of 2.5mol/L, a phosphate buffer with pH 7 present at a concentration of 0.02 mol/L and hydrogen peroxide present at a concentration of 1%.
3. Putting the bioprosthetic valve treated in step 2 into a solution for 30 hours, wherein the solution comprising Ethanol-50% and indometacin present at a concentration of 100mg/L.

### Example 3

1. Soaking the bioprosthetic valve in Ethanol-50% solution for 30 days.
2. Putting the bioprosthetic valve treated in step 1 into a solution for 96 hours, wherein the solution comprises a polyethylene glycol have a molecular weight of 8000D present at a concentration of 15%, sodium chloride present at a concentration of 4.5mol/L, a phosphate buffer with pH 7 present at a concentration of 0.1 mol/L and hydrogen peroxide present at a concentration of 2%.
3. Putting the bioprosthetic valve treated in step 2 into a solution for 30 hours, wherein the solution comprises Ethanol-50% and indometacin present at a concentration of 200mg/L.
4. Putting the bioprosthetic valve treated in step 3 into a solution for 24 hours, where the solution comprises Ethanol-50% and enoxaparin present at a concentration of 1000 IU/ml.

### Example 4

1. Soaking the bioprosthetic valve in 0.625% glutaraldehyde solution for 10 days, obtaining the bioprosthetic valve (bovine pericardium biovalve) treated with glutaraldehyde, used as control.
2. Sample transplantation: removing the bioprosthetic valves from Example 1 to 3 and control bioprosthetic valve 35 days after being subcutaneously transplanted into rats.
3. Sample formulation: removing the bioprosthetic valve, which was rinsed to remove blood, then placed in an oven, dried at 90 °C x 40 hours, after standard and accurate weighment of dry weight, it was heated and decomposed by using concentrated nitric acid diluted with 1:1 volume ratio, fully oxidized by hydrogen peroxide after cooling, then made up to the mark with water, ready for sample feeding.
4. Standard formulation: placing the precisely weighed calcium standard solution in a volumetric flask, an appropriate amount of concentrated nitric acid diluted with 1:1 volume ratio was added, followed by adding hydrogen peroxide equivalent to the sample, then made up to the mark with water, ready for sample feeding.
5. Determination method of calcium content: obtaining calcium content of the bioprosthetic valve from the change in absorption value during the burning process of the decomposition fluid of the bioprosthetic valve by atomic absorption spectrophotometer (AAS).

### AAS parameters:

| Instrument Type | Zeeman |
|---|---|
| Conc. Units | µg/L |
| Instrument Mode | Absorbance |
| Sampling Mode | Auto normal |
| Calibration Algorithm | Linear |
| Calibration Mode | Concentration |
| Measurement Mode | Peak Area |
| Replicates Standard | 3 |
| Replicates Sample | 3 |
| Expansion Factor | 1 |
| Minimum Reading | Disabled |
| Smoothing | 7 point |
| Conc. Dec. Places | 4 |
| Wavelength | 422.7 nm |
| Slit Width | 0.5 nm |
| Lamp Current | 10.0 mA |
| Background Correction | BC open |
| Sample Volume | 10µL |
| Total Volume | 15µL |

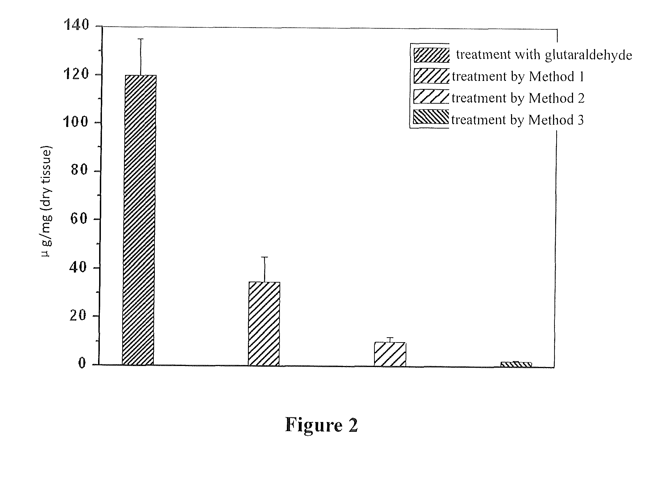

The particular values of calcium content are shown in Figure 2.

The above description of the examples is merely used to help understand the core idea of the present invention. It should be noted that for those of ordinary skill in the art, a number of improvements and modifications can also be made to the method of the present invention without departing from the principles of the invention, however, these improvements and modifications fall within the claimed scope of the invention.

## Claims

1. A method for treating an animal-derived collagen fibre material, comprising a step of treating the animal-derived collagen fibre material with a salt solution containing a polyethylene glycol and an oxidant.

2. The method according to claim 1, wherein, the salt solution has a concentration of at least 2.5mol/L.

3. The method according to claim 1 or 2, wherein, the oxidant is hydrogen peroxide.

4. The method according to any of the preceeding claims, wherein, the animal-derived collagen fibre material is selected from the group consisting of tendon, cornea, cardiac valve, ligament, skin and diaphragm.

5. The method according to claim 4, wherein, the animal-derived collagen fibre material is a bioprosthetic valve.

6. The method according to any of the preceeding claims, comprising the steps of:
triming tissue material removed from an animal body in a physiologicalsaline and soaking the trimed material in a stabilizing solution at 0 to 10°C, wherein the stabilizing solution is a physiologicalsaline or Ethanol-30-70%, and wherein the soaking is carried out not more than 48 hours when using the physiologicalsaline and not more than 30 days when using the Ethanol-30-70%;
treating thus obtained tissure material with a Solution I for 2 to 2400 hours at 0 to 10 °C, wherein said Solution I comprises a polyethylene glycol having a molecular weight of 2000D to 20000D present at a concentration of 1% to 15%, sodium chloride present at a concentration of 2.5mol/L to 4.5mol/L, a phosphate buffer with pH 6.5 to 7 present at a concentration of 0.02 to 0.1 mol/L and the oxidant present at a concentration of 0.1% to 2%;
removing the tissue material from Solution I and washing it with an aqueous solution of low concentration ethanol to remove residue of the Solution I.

7. The method according to claim 6, further comprising:
after treating with the Solution I and washing with the aqueous solution of ethanol, treating the tissue material with a Solution II comprising Ethanol-30%-70% and an anti-inflammatory drug present at a concentration of 10 to 200mg/L;
optionally soaking the tissue material in a Solution III comprising Ethanol-30-70% and an anticoagulant present at a concentration of 100 to 1000 IU/ml; and
finally washing the tissue material with a physiological saline and submerging and storing the tissue material in Ethanol-30-70% at 0 to 6°C.

8. The method according to claim 6, further comprising:
after treating with the Solution I and washing with the aqueous solution of ethanol, soaking the tissue material in a Solution IV comprising Ethanol-30-70%, an anti-inflammatory drug present at a concentration of 10 to 200mg/L and an anticoagulant present at a concentration of 100 to 1000 IU/ml; and
finally washing the tissue material with a physiological saline and submerging and storing the tissue material in Ethanol-30-70% at 0 to 6°C.

9. The method according to claim 7 or 8, wherein, the anti-inflammatory drug is selected from the group consisting of indometacin, dexamethasone acetate, dexamethasone, rapamycin, prednisone, prednisone acetate, antiflamison, ibuprofen piconol, ibuprofen and bufexamac, while the anticoagulant is selected from the group consisting of heparin, enoxaparin and hirudin.
